# EUROPEAN PATENT APPLICATION

(11) **EP 0 865 779 A1**
(43) Date of publication of application: **23.09.1998**
(21) Application number: 98302035.5
(22) Date of filing: 18.03.1998
(51) Int. Cl.: A61F 5/448

(54) **Orifice couplings, parts therefor, and methods of production**

(30) Priority: 21.03.1997 GB 9705905
(71) Applicant: BRISTOL-MYERS SQUIBB COMPANY, Princeton, NJ 08543-4000 (US)
(72) Inventor: Steer, Peter Leslie, East Grinstead, Sussex, RH10 4JZ (GB)
(74) Representative: Holmes, Miles Keeton

(57) **Abstract**

An orifice coupling (e.g. an ostomy coupling) comprises a first bodyside coupling member (10), a second bagside coupling member (12) and a locking ring (14). One of the coupling members (10) carries a deflectable sealing strip (34); that coupling member consists of a structural outer part (36) of a relatively hard or firm plastics material, and an inner part (38) carrying the seal (34) and made of a relatively soft or deformable plastics material. The two parts may be integrally moulded, for example by a 2-shot moulding process.

## Description

This invention relates to orifice couplings and parts or elements therefor. The invention is particularly suitable for use in the field of ostomy couplings, but it is not limited exclusively to that field. For example, the invention may also find applications in incontinence couplings, or woundcare couplings.

Ostomy couplings are used to connect and disconnect a bag for receiving a stomal discharge to and from a medical grade adhesive pad or base which is applied to the peristomal area of the skin of the wearer.

Many designs of ostomy coupling are known. In particular, EP-A-0737456, EP-A-0737457, EP-A-0737458, EP-A-0737459 and EP-A-0737460 describe couplings having mechanical locking arrangements for locking the body-side and bag-side coupling elements together. The arrangements each use a springy locking ring from which locking tabs project radially inwardly. The coupling elements can be assembled with a simple snap together action, during which the tabs are displaced outwardly and then snap back inwardly to hold the coupling elements together. To release the coupling, the locking ring is manipulated to displace the tabs outwardly, which allows the coupling elements to be separated.

In contrast to the prior art arrangements in which each coupling element is made of a single material, one aspect of the present invention is to make a first coupling element of an orifice coupling of first and second plastics materials. A first part or portion of the coupling element can be made of a first relatively firm or stiff material, and a second part or portion can be made of a second relatively soft or deformable material.

In one preferred aspect, the invention provides an orifice coupling for establishing a connection to an orifice in a body or in a bag, the coupling comprising a first coupling element and a second coupling element releasably securable together, the first coupling element comprising a first part or portion of a first relatively rigid plastics material integrally moulded with a second part or portion of a second relatively deformable plastics material.

In another preferred aspect, the invention provides an orifice coupling comprising first and second releasably securable coupling elements surrounding an orifice, the first coupling element comprising a first part or portion of a first relatively rigid plastics material, and a second part or portion of a second relatively deformable plastics material, the second part or portion comprising a deflectable sealing strip for forming a seal against the second coupling member.

In yet a further preferred aspect, the invention provides an orifice coupling comprising first and second coupling members securable together by means of a deformable locking ring carried in one of the coupling elements for locking engagement with the other coupling element, the first coupling element comprising a first part or region of a first relatively rigid plastics material, and a second part or region of a first relatively deformable plastics material, the first part or region comprising means for mounting or engaging the locking ring.

In another aspect, the invention provides an orifice coupling for establishing a connection to an orifice in a person's body or in a bag, the coupling comprising a first coupling member and a second coupling member surrounding orifice and releasably securable together, the first coupling member comprising a first part or portion of a first relatively rigid plastics material integrally moulded with a second part or portion of a second relatively deformable plastics material, the second part or portion comprising a sealing element for sealing against the second coupling member.

In another aspect, the invention provides an orifice coupling for establishing a connection to an orifice in a person's body or in a bag, comprising first and second coupling members surrounding the orifice and releasable locking means for mechanically securing the coupling members together, the first coupling member comprising a first part or portion of a first relatively rigid plastics material for mounting or engaging the locking means, and a second part or portion of a second relatively deformable plastics material, the second part or portion comprising a deflectable sealing strip distinct from the locking means for forming a seal against the second coupling member.

In yet a further aspect, the invention provides an orifice coupling for establishing a connection to an orifice in a person's body or in a bag, comprising first and second coupling members surrounding the orifice and securable together by means of a deformable locking ring carried by one of the coupling members for locking engagement with the other coupling member, the first coupling member comprising a first part or region of a first relatively rigid plastics material, and a second part or region of a second relatively deformable plastics material, the first part or region comprising means for mounting or engaging the locking ring, and the second part of region comprising a sealing element for forming a seal with the second coupling member.

In a further closely related aspect, the invention provides a coupling member for use in an orifice coupling according to any of the preceding aspects, the coupling element comprising a said first part or portion of a first relatively rigid plastics material, and a said second part or portion of a second relatively deformable plastics material integrally moulded with the first part or portion.

With the invention in its various aspects, the limitations of using a single material for these types of coupling member, particularly an ostomy coupling member, have been appreciated for the first time.

An example of such limitation is the requirement for the coupling members to be relatively stiff in order to achieve reliable and secure mechanical support and engagement with each other, and the conflicting requirement for a pliable seal which is easily deflectable so as not to increase the insertion force needed to assemble the coupling.

For example, in the prior art arrangements discussed above, it is advantageous in one respect to use stiff plastics material, to produce a positive snap together coupling action with a definitive "click" when the coupling is properly assembled. However, increasing the stiffness of the plastic material also increases the stiffness of the deflectable seal, which may reduce the effectiveness of the seal and increase the insertion force required to insert the other coupling member past the seal. Of course, reducing the stiffness of the material would benefit the seal characteristics and reduce the insertion force required to assemble the coupling. However, a reduction in the stiffness could be detrimental to the mechanical locking arrangement, and may fail to produce the desired snap-together action with a positive "click"

With the invention, the above limitations can be alleviated. It is believed that this has not been appreciated in the art hitherto because of a number of reasons, which include the small size of the components, and the desirability of an integrally formed "single-part" coupling member.

Although the invention is particularly suitable for use with a three-part coupling (as illustrated in the preferred embodiments), the invention is not limited thereto and can be used equally advantageously with, for example, a two-piece coupling.

In a related aspect, the invention provides a method of producing a coupling or coupling member as aforesaid, the method comprising integrally moulding a first part or portion and a second part or portion of a first coupling member, the first part or portion being of a first relatively rigid plastics material, and the second part or portion being of a second relatively deformable plastics material.

One preferred technique for integrally moulding the first and second parts or portions of the coupling member is by a so-called 2-shot moulding process (know per se). In such a process, one of the portions is moulded first, after which the other portion is moulded in direct contact with the first portion. The second portion can either be moulded before the first portion has set, to achieve a chemical bond between the two portions, or it can be moulded after the first portion has set, in which case a mechanical fixing or keying arrangement would be required between the two portions.

Although know per se, 2-shot moulding has not hitherto been used to form orifice couplings of the present types. In particular, the advantages achievable by such moulding are not appreciated in the prior art.

The present invention will be understood in more detail from the following description of preferred embodiments, presented by way of example only, and illustrated in the accompanying drawings, in which:-
Figure 1 is a horizontal sectional view of the bodyside half of a first embodiment of ostomy coupling;
Figure 2 is a vertical section through the bodyside coupling element in isolation;
Figure 3 is a vertical sectional view of the bodyside and bagside coupling members in an assembled condition;
Figure 4 is a plan view of the bagside coupling member;
Figure 5 is a vertical sectional view of a second embodiment of ostomy coupling;
Figure 6 is a plan view of the bagside coupling member of the second embodiment;
Figure 7 is a sectional view showing a detail of a first modification of the bagside coupling member design; and
Figure 8 is a sectional view showing a detail of a second modification of the bagside coupling member design.

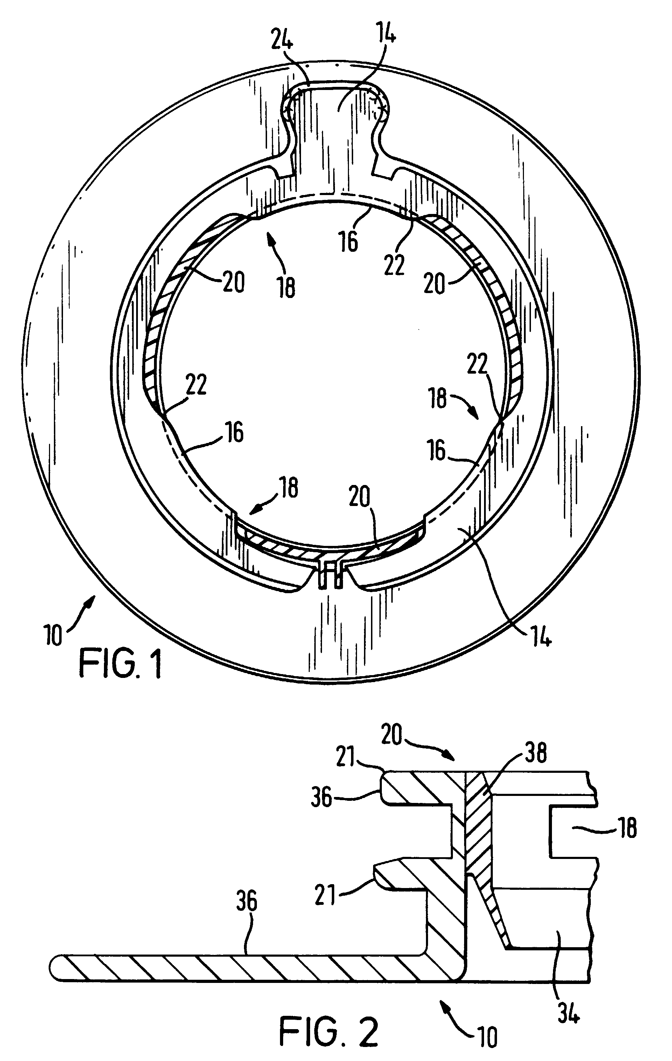

Referring to Figs. 1 to 4, the ostomy coupling consists of a first coupling member 10, a second coupling member 12 and a split locking ring 14. In this embodiment, the first coupling member 10 is the bodyside member, and the second coupling member 12 is the bagside member, although in other embodiments the roles could be reversed.

Except for the important structural differences described further below, the coupling is very similar to that described in EP-A-0737458. The reader is referred to that document for detailed description of the operation of the coupling, and the manner in which the coupling members are attached to a wearer's body and to an ostomy bag.

The locking ring 14 is mounted on the first coupling member 10, and comprises inwardly directed locking tabs 16 which project through corresponding slots 18 in a cylindrical wall 20 of the first coupling member. The ends of the tabs 16 are formed with circumferential ramp surfaces 22 which slide against the end walls of the slots 18 when the locking ring 14 is rotated by means of its handle 24 to force the tabs 16 radially outwardly. The split in the locking ring enables the "limbs" of the ring to move apart to expand the ring. The springiness of the ring material urges the tabs 16 to return to their radially inwardly projecting positions when the ring 14 is rotated back to its normal position (as shown in Fig. 1). The wall 20 is formed with projecting annular ribs 21 which define a channel shaped outer profile in which the locking ring 14 is retained, in use.

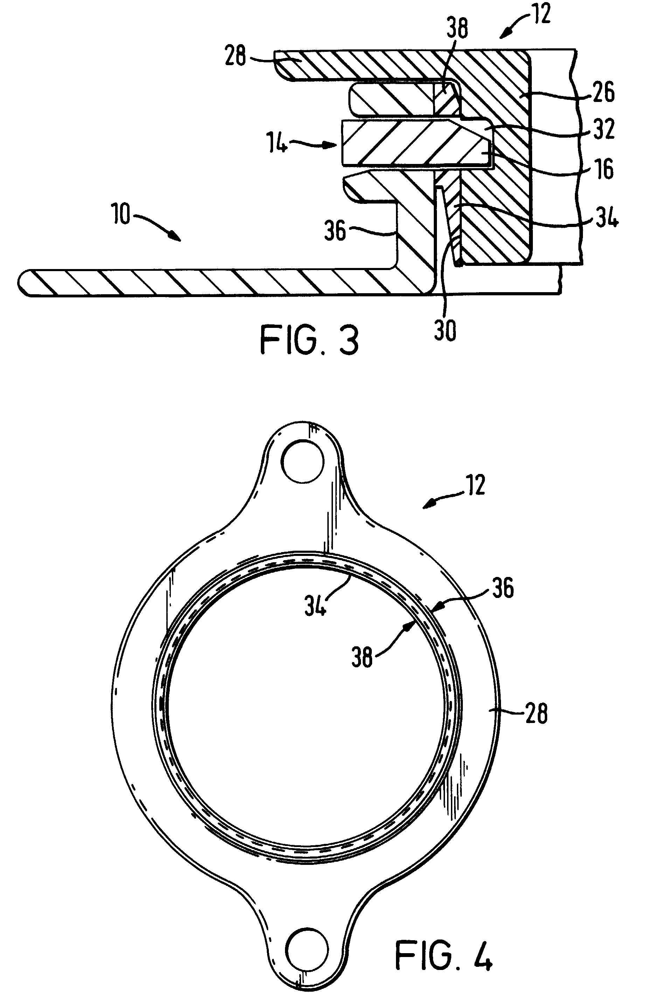

The second coupling member 12 (Fig. 3) consists of a cylindrical wall 26 and a flange 28 to which the bag is attached. The cylindrical wall 26 is dimensioned to fit inside the cylindrical wall 20 of the first coupling member. Its outer face has an engagement profile which includes a lead-in taper 30 behind which is an annular recess 32 for receiving the locking tabs 16 of the locking ring 14.

Below the slots 18, the cylindrical wall 20 (Figs. 1 and 2) of the first coupling element 10 carries a deflectable annular seal strip or wiper 34 for forming a seal against the cylindrical wall 26 of the second coupling member. The function of the seal strip is to prevent leakage and to take up any minor tolerance variations which may have arisen in the manufacturing of the first and second members 10 and 12, or as a result of attachment to a wearer.

In contrast to the arrangement described in EP-A-0737458, in the present embodiment, the first coupling member 12, or more particularly its cylindrical wall 20, is made of two materials. As best seen in Fig. 3, it consists of an outer structural moulding 36 of a relatively rigid (hard or firm) plastics material, and an inner liner 38 of a relatively deformable (soft) plastics material and carrying the seal 34. As an example, the outer moulding 36 may be of a high density polyethylene (HDPE) or a polypropylene; the inner liner 38 may be of a low density polyethylene (LDPE) or an elastomer.

The hard outer moulding 36 provides the component with the rigidity required to support the ostomy bag and the bagside coupling when attached thereto. Additionally, it prevents deformation of the cylindrical wall 20 which might otherwise permit movement of the locking ring 14 out of its correct plane, and reduce the effectiveness and positive feel of the snap fit coupling. The snap fit action is provided by the taper surface 30 which (upon coupling of the two members) urges the tabs 16 outwardly until the tabs 16 snap back to locate in the annular recess 32.

The soft material forming the seal wiper 34 provides a pliable seal able to adapt easily to any imperfections in the cylindrical wall 26 of the second coupling member 12, and establish and maintain a reliable seal. Furthermore, it will be appreciated that one of the significant factors affecting the insertion force required to assemble the coupling is the force necessary to deflect and expand the seal wiper 34 which naturally has an inner diameter slightly smaller than the outer diameter of the cylindrical wall 26 against which it seals. In prior art designs, the seal wiper was made of the same relatively hard material as the remainder of the first coupling member, which therefore offered quite a high resistance to the insertion of the second coupling member into the first coupling member. By using a seal wiper 34 of soft material as in the present embodiment, there is much less resistance to deflection of the wiper 34, and this further enhances the desirable low insertion force characteristics of the coupling.

The first coupling member 10 may be produced by any convenient process. With the illustrated structure, so called "two shot" moulding may be used. In such a process, a first part, for example, the outer part 36 of relatively hard plastics, is moulded as a first step. Thereafter, before the plastics material of the first part has set, the other part, in this case the inner part 38 of soft plastics, is moulded directly in contact with the plastics of the first part. The two plastics materials need to be of chemically compatible materials to form a chemical bond as the two materials set. The two parts can either be moulded one after the other in the same mould by using different mould parts, or the first part can be transferred to a separate mould for moulding the second part thereon.

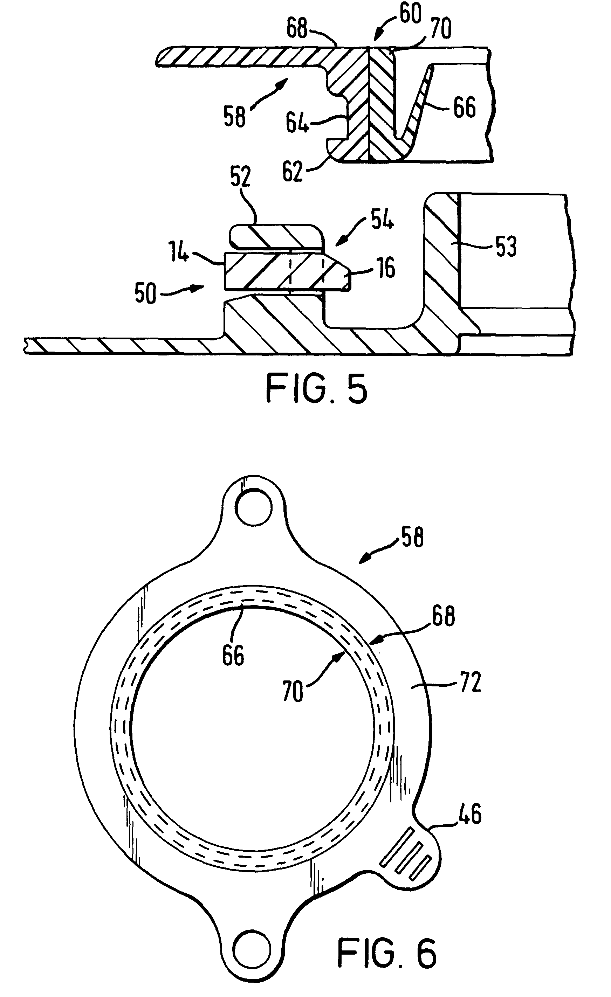

Figs. 5 and 6 illustrate a further embodiment which uses the same type of locking ring as the first embodiment, but includes modified designs of bodyside and bagside coupling members.

Referring to Figs. 5 and 6, a first (bodyside) coupling member 50 comprises dual cylindrical walls 52 and 53 surrounded by the split locking ring 14. The outer cylindrical wall 52 has slots 54 therethrough similar to the slots 18 of the first embodiment, through which the tabs 16 of the locking ring project.

A second (bagside) coupling member 58 comprises a cylindrical wall 60 dimensioned to fit between the cylindrical walls 52 and 53 of the first coupling member 50. The outer face of the cylindrical wall 60 has a lead-in taper surface 62, and an annular engagement recess 64 similar to the recess 32 of the first embodiment. The inner face of the cylindrical wall carries a deflectable outwardly directed annular seal strip or wiper 66 which is similar to the inwardly directed seal wiper 34 of the first embodiment. In use, when the second coupling member 58 is inserted into the first coupling member 50, the couplings form a snap fit as in the preferred embodiment. The seal wiper 66 engages against the outer surface of the inner cylindrical wall 53 of the first coupling member 50.

In this embodiment, the second coupling element 58, instead of the first coupling element, is made of two materials. The second coupling element 58 comprises a outer moulding 68 of relatively hard or firm plastics, and an inner liner 70 (including the seal wiper 56) of relatively soft material.

The use of the soft material for the deflectable seal provides the same advantages as in the first embodiment, namely the reduction of the insertion force required to deflect the seal, and an improvement in sealing engagement. Furthermore, the use of relatively rigid material for the outer part 68 can ensure that it is retained reliably by the locking tabs 16 of the locking ring 14, and can provide a positive, solid snap-fit action when the second coupling member 58 is pressed into the space between the two cylindrical walls 52 and 53 of the first coupling member 50 to assemble the ostomy coupling. The snap-fit action is provided by the taper surface 62 which urges the tabs 16 outwardly until the tabs 16 snap back to locate in the annular recess 64. It will be appreciated that to achieve a desirable low insertion force coupling, the locking ring should be easily deformable so that it will expand in response to application of a modest insertion force. Such an easily deformable ring 14 will only grip the recess 64 weakly, and this increases the importance of the outer moulding 68 being rigid to establish a reliable locking action between the tabs 16 and the recess 64.

The second coupling member may be produced using the same 2-shot moulding operation described above for the first embodiment.

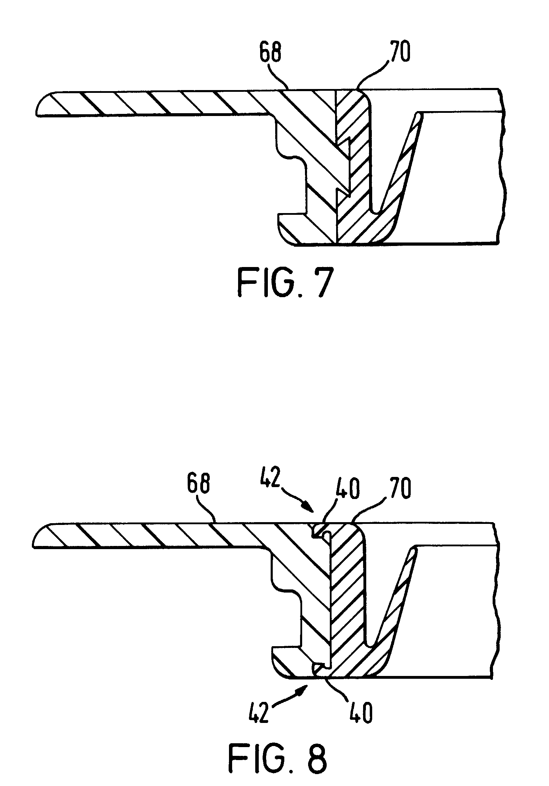

In either embodiment, it is also possible to allow the material of the first part (36 or 68) of the moulding to set before the second part (38 or 70) is moulded. In such case, a mechanical fixing or keying arrangement would be required, such as that illustrated in Fig. 7. The mechanical fixing arrangement provides a mechanical interlock between the first and second parts, instead of (or in addition to) a chemical bond. In the example illustrated in Fig. 7, the first part (68) is moulded with a dovetailed projection; when the second part (70) is moulded later, the material of the second part flows around the projection to complete the interlock. It will be appreciated that the dovetailed projection could be replaced with a dovetailed groove, or any other mechanical keying profile into or around which the second part can be moulded. It will also be appreciated that mechanical keying can be employed with the previous "two shot" chemical bond to reinforce the bond between the first and second parts.

As a further alternative, the first and second parts (36 and 38; 68 and 70) could be moulded independently as two distinct items, and then assembled to form the respective coupling member. For example, referring to Fig. 8, the liner 70 could be moulded for snap together engagement with the outer part 68. In Fig. 8, the liner 70 is moulded with rimmed flanges 40 defining a C-shape outer profile. The outer part 68 is formed with complementary recesses 42 into which the rimmed flanges engage with a snap fit when the liner 70 is pressed into the outer part 68.

Referring to Fig. 6, a radially projecting finger grip tab 46 may be formed on the flange 72 of the second coupling member 12. The tab 46 enables a wearer to grip the flange 72 directly during assembly and disassembly of the coupling, when an ostomy bag is to be fitted or removed. The tab 46 may be of the same material as the outer moulding 68, or it may be of a soft flexible material so that it can be bent forward to enable a user to obtain a better grip of the tab 46. The tab material may be the same as that of the liner 70, or it may be a different soft material.

In this embodiment, the tab 46 is of a different colour material from the flange 72, for ease of identification. The tab 46 may, for example, be formed using a "two shot" moulding process as described above.

The embodiments illustrated above are based on the prior art design described in EP-A-0737458. However, it will be appreciated that the principles may be used with any ostomy coupling, particularly (although not exclusively) couplings which employ manual locking arrangements. Particular reference is made to the arrangements in EP-A-0737456, EP-A-0737457, EP-A-0737459 and EP-A-0737460

Although the invention has been illustrated in terms of an ostomy coupling, the skilled man will appreciate that the principles of the invention may also be used in other fields, such as woundcare and incontinence couplings.

It will be appreciated that the use of a relatively soft material and a relatively hard material can provide significant advantages over conventional designs which are moulded of a single material. Although the illustrated embodiments employ a deflectable seal, it will be appreciated that this is merely illustrative, and that a compressible o-ring seal, for example of compressible material, could be used instead. The o-ring seal may be made of a relatively soft material, and carried by or moulded with, the coupling member made of a relatively stiff or rigid material. Similarly, although the illustrated embodiments employ a radial type seal (i.e. a seal which exerts a generally radial sealing force between the coupling members), other types of seal, such as an axially compressible or deflectable seal may be used. The principles of the invention would benefit all such variations of seal.

## Claims

1. An orifice coupling for establishing a connection to an orifice in a body or in a bag, the coupling comprising a first coupling member and a second coupling member releasably securable together, the first coupling member comprising a first part or portion of a first relatively rigid plastics material integrally moulded with a second part or portion of a second relatively deformable plastics material.

2. A coupling according to claim 1, wherein the second part or portion of the first coupling member comprises a sealing member for sealing against the second coupling member.

3. A coupling according to claim 2, wherein the sealing member comprises a deflectable strip.

4. An orifice coupling comprising first and second releasably securable coupling members surrounding an orifice, the first coupling member comprising a first part or portion of a first relatively rigid plastics material, and a second part or portion of a second relatively deformable plastics material, the second part or portion comprising a deflectable sealing strip for forming a seal against the second coupling member.

5. A coupling according to claim 3 or 4, wherein the sealing strip is generally circular, or is generally annular.

6. A coupling according to claim 5, wherein the sealing strip is generally frusto conical.

7. A coupling according to claim 5 or 6, wherein the sealing strip has an inner dimension which is slightly smaller than an outer dimension of the second coupling member in the region of the second coupling member in which a seal is established.

8. A coupling according to any preceding claim, wherein the first part or portion of the first coupling member is load bearing.

9. A coupling according to any preceding claim, further comprising locking means for mechanically securing the first and second coupling members together.

10. A coupling according to claim 9, wherein the locking means comprises a deformable locking ring carried on one of the coupling members for locking engagement with the other coupling member, the first part or portion of the first coupling member comprising means for mounting or engaging the locking ring.

11. An orifice coupling comprising first and second coupling members securable together by means of a deformable locking ring carried in one of the coupling members for locking engagement with the other coupling member, the first coupling member comprising a first part or region of a first relatively rigid plastics material, and a second part or region of a first relatively deformable plastics material, the first part or region comprising means for mounting or engaging the locking ring.

12. A coupling according to claim 10 or 11, wherein the locking ring is carried on the first coupling member and the first part region of the first coupling member comprises means for captively engaging the locking ring.

13. A coupling according to claim 12, wherein said captive engagement means comprise one or more apertures in a wall of the first part or region of the first coupling member, for receiving projections of the locking ring.

14. A coupling according to claim 11, wherein the locking ring is carried on the second coupling member, and the first part or region comprises a recess for receiving an engagement part of the locking ring.

15. A coupling according to any preceding claim, wherein at least one of the first and second coupling members comprises a radially projecting mounting flange.

16. A coupling according to any preceding claim, wherein the or a second part or portion comprises a finger tab projecting from the first part or portion of the coupling member.

17. A coupling according to any preceding claim, wherein the coupling is an ostomy coupling.

18. A coupling according to claim 17, wherein the first part or portion and the second part or portion of the first coupling member are formed by 2-shot moulding.

19. A coupling member for use in an orifice coupling according to any preceding claim, the coupling member comprising a first part or portion of a first relatively rigid plastics material, and a second part or portion of a second relatively deformable plastics material.

20. A method of producing a coupling or coupling member as defined in any preceding claim, the method comprising integrally moulding a first part or portion and a second part or portion of a first coupling member, the first part or being of a first relatively rigid plastics material, and the second part or portion being of a second relatively deformable plastics material.

21. A method according to claim 20, wherein the step of moulding comprises moulding the first and second parts or portions by 2-shot moulding.

22. A method according to claim 20 or 21, wherein the first and second parts or portions comprise mutually interlocking engagement profiles.

23. A method according to any preceding claim, wherein the first part or portion is moulded prior to the second part or portion.

24. An orifice coupling for establishing a connection to an orifice in a person's body or in a bag, the coupling comprising a first coupling member and a second coupling member surrounding orifice and releasably securable together, the first coupling member comprising a first part or portion of a first relatively rigid plastics material integrally moulded with a second part or portion of a second relatively deformable plastics material, the second part or portion comprising a sealing element for sealing against the second coupling member.

25. An orifice coupling for establishing a connection to an orifice in a person's body or in a bag, comprising first and second coupling members surrounding the orifice and releasable locking means for mechanically securing the coupling members together, the first coupling member comprising a first part or portion of a first relatively rigid plastics material for mounting or engaging the locking means, and a second part or portion of a second relatively deformable plastics material, the second part or portion comprising a deflectable sealing strip distinct from the locking means for forming a seal against the second coupling member.

26. An orifice coupling for establishing a connection to an orifice in a person's body or in a bag, comprising first and second coupling members surrounding the orifice and securable together by means of a deformable locking ring carried by one of the coupling members for locking engagement with the other coupling member, the first coupling member comprising a first part or region of a first relatively rigid plastics material, and a second part or region of a second relatively deformable plastics material, the first part or region comprising means for mounting or engaging the locking ring, and the second part of region comprising a sealing element for forming a seal with the second coupling member.

27. A method of producing a coupling or coupling member as defined in any preceding claim, the method comprising integrally moulding a first part or portion and a second part or portion of a first coupling member, the first part or portion being of a first relatively rigid plastics material, and the second part or portion being of a second relatively deformable plastics material.
